(19) Europäisches Patentamt
European Patent Office
Office européen des brevets

(11) Publication number: **0 002 942**
**B1**

(12) # EUROPEAN PATENT SPECIFICATION

(45) Date of publication of patent specification: **12.08.81**

(51) Int. Cl.³: **C 07 C 97/07**

(21) Application number: **78300885.7**

(22) Date of filing: **21.12.78**

(54) **Process for producing 3-amino-2-cyclohexenone.**

(30) Priority: **28.12.77 JP 157326/77**

(43) Date of publication of application:
**11.07.79 Bulletin 79/14**

(45) Publication of the grant of the European patent:
**12.08.81 Bulletin 81/32**

(84) Designated Contracting States:
**DE FR GB NL**

(56) References cited:
**DE - A - 2 144 169**
**DE - A - 2 144 170**
**FR - A - 2 018 254**

(73) Proprietor: **MITSUI PETROCHEMICAL INDUSTRIES, LTD.**
**2-5, Kasumigaseki 3-chome Chiyoda-ku**
**Tokyo 100 (JP)**

(72) Inventor: **Kiso, Yoshihisa**
**2-3, Muronoki-cho 1-chome**
**Iwakuni-shi Yamaguchi-ken (JP)**
Inventor: **Saeki, Kenji**
**2-7, Misono 1-chome**
**Ohtake-shi Hiroshima-ken (JP)**

(74) Representative: **Myerscough, Philip Boyd et al,**
**J.A.Kemp & Co. 14, South Square Gray's Inn**
**London, WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 002 942 B1

Process for producing 3-amino-2-cyclohexenone

This invention relates to a process for producing 3-amino-2-cyclohexenone which is useful as an intermediate for the production of m-aminophenol, cyclohexane-1,3-dione, resorcinol, etc. Specifically, it relates to a process for producing 3-amino-2-cyclohexenone in superior conversion and selectivity by heat-cyclization of 5-oxohexanenitrile.

More specifically, the invention relates to a process for producing 3-amino-2-cyclohexenone of the formula

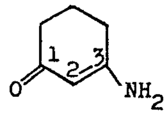

which comprises heating 5-oxohexanenitrile of the formula

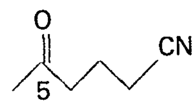

at a temperature of 160°C to 220°C in the presence of a basic catalyst in an inert organic polar solvent.

Japanese Laid-Open Patent Publication No. 34859/73 (published on May 22, 1973; corresponding to German OLS 2144169 and 2144170 published on March 8, 1973) discloses a process for preparing substituted cyclohexen-2-ones which comprises reacting a cyanoethylated ketone of the formula

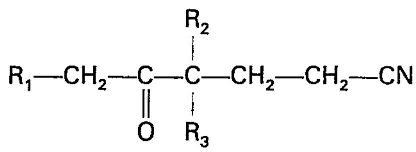

wherein $R_1$ represents hydrogen or alkyl of 1 to 5, preferably 1 to 3, carbon atoms, and $R_2$ and $R_3$ are identical or different and represent hydrogen, alkyl of 1 to 5, preferably 1 to 3, carbon atoms, or $\beta$-cyanoethyl,

at a temperature of 20 to 150°C in the presence of a basic catalyst such as an alkali metal hydroxide in a polar solvent.

This Japanese Publication does not give a specific disclosure about the production of 3-amino-2-cyclohexenone from 5-oxohexanenitrile which corresponds to a compound of the above formula in which all of $R_1$, $R_2$ and $R_3$ are hydrogen. All the working examples in this publication are directed to the production of 3-amino-2-cyclohexenones from 4-substituted 5-oxohexanenitrile in which $R_2$ or $R_3$ is a substituent other than hydrogen, such as alkyl or $\beta$-cyanoethyl, and the reaction temperature is limited to about 20 to 150°C.

The present inventors attempted the production of the 3-amino-2-cyclohexenone, which is useful as an intermediate for various useful compounds including m-aminophenol, cyclohexane-1,3-dione and resorcinol, by the cyclization of 5-oxohexanenitrile. This attempt led to the discovery that at a temperature of 20 to 150°C recommended by the prior patent literature, the cyclization of 5-oxohexanenitrile with an unsubstituted 4-position to 3-amino-2-cyclohexenone gives much worse results than the cyclization of the aforesaid 4-substituted compound. Accordingly, the inventors worked on the cyclization reactivity of the 5-oxohexanenitrile, and found that at the reaction temperatures recommended by the prior art, the reactivity of the 5-oxohexanenitrile with the unsubstituted 4-position is low, and the selectivity of the starting compound for 6-methyl-3,4-dihydro-2-pyridone increases while its selectivity for the desired 3-amino-2-cyclohexenone and the yield of the 3-amino-2-cyclohexenone are low.

The inventors furthered their investigations in order to overcome the technical disadvantage associated with the above 5-oxohexanenitrile which is not specifically disclosed by the prior art. It has consequently been found that within a temperature range of 160°C to 220°C, preferably 170°C to 200°C, which exceeds the upper limit (150°C) of the reaction temperatures disclosed by the prior art, the conversion of 5-oxohexanenitrile and its selectivity for 3-amino-2-cyclohexenone increase critically, and at temperatures below the lower limit of the above temperature range (i.e., the temperatures suggested heretofore) and above the upper limit of the above temperature range, the selectivity for the by-product 6-methyl-3,4-dihydro-2-pyridone abruptly increases and the selectivity for the desired 3-amino-2-cyclohexenone and its yield are reduced markedly.

It is an object of this invention therefore to provide a process for producing 3-amino-2-cyclohexenone with commercial advantage by the heat cyclization of 5-oxohexanenitrile.

The above and other objects and advantages of the invention will become apparent from the following description.

According to the process of this invention, 3-amino-2-cyclohexenone of the formula given hereinabove can be produced in high conversions and selectivities by heating 5-oxohexanenitrile of the formula given hereinabove at 160°C to 220°C, preferably 170°C to 200°C, in the presence of a basic catalyst in an inert organic polar solvent to cyclize the 5-oxohexanenitrile.

Specific examples of the solvent used in this process include alcohols containing 1 to 6 carbon atoms such as methanol, ethanol, n-propanol, isopropanol, n-butanol, sec-butanol, tert-butanol, pentanol, tert-amyl-alcohol and hexanol; ethers containing 4 to 14 carbon atoms such as diethyl ether, tetrahydrofuran, dioxane, diglyme and triethylene glycol dimethyl ether; nitriles containing 1 to 3 carbon atoms such as acetonitrile and propionitrile; amines containing 3 to 8 carbon atoms such as trimethylamine, triethylamine, pyridine and $\alpha$-picoline; dialkylformamides with the alkyl group containing 1 or 2 carbon atoms such as dimethylformamide and diethyl formamide; phosphoric acid amides such as hexamethylphosphoric triamide; and sulfoxides such as dimethyl sulfoxide. Of these, tertiary alcohols, especially tert-butanol, are preferred. Mixtures of two or more of these organic polar solvents, and mixtures of them with organic non-polar solvents can also be used. Examples of the organic non-polar solvents are aliphatic hydrocarbons such as hexane, heptane, octane, nonane and decane; and aromatic hydrocarbons, preferably of 6 to 10 carbon atoms, such as benzene, toluene and p-xylene. The amount of the organic polar solvent can be used in an amount such that the weight ratio of it to the 5-oxohexanenitrile is from 1:1 to 1000:1, preferably from 5:1 to 200:1.

Specific examples of the basic catalyst used in the above process are alkali metal hydroxides such as potassium hydroxide and sodium hydroxide; alkali metal alkoxides such as potassium methoxide, potassium ethoxide, potassium propoxides and potassium butoxides and the corresponding sodium alkoxides; alkali metal amides such as sodium amide and potassium amide; alkali metal hydrides such as sodium hydride; and alkali metals such as sodium and potassium. Of these basic catalysts, sodium hydroxide, potassium hydroxide, potassium alkoxides, and sodium alkoxides are preferred. Potassium hydroxide and potassium alkoxides are especially preferred. The amount of the basic catalyst can be varied widely. Usually, it is 0.001 to 1 mole, preferably 0.005 to 0.2 moles, per mole of the starting 5-oxohexanenitrile.

The heat cyclization reaction is carried out at a temperature of 160 to 220°C, preferably 170 to 200°C. If the heating temperature is lower than about 160°C, the conversion of the 5-oxohexanenitrile decreases, and its selectivity for 6-methyl-3,4-dihydro-2-pyridone, a by-product, increases abruptly, thus causing a reduction in the selectivity for 3-amino-2-cyclohexanone and its yield. On the other hand, if the heating temperature exceeds about 220°C, the selectivity for the by-product 6-methyl-3,4-dihydro-2-pyridone increases, and 3-amino-2-cyclohexenone which has been formed may be consumed by a secondary reaction. Hence, the selectivity for 3-amino-2-cyclohexenone and its yield decrease. By maintaining the heating temperature at 170 to 200°C, the selectivity for 3-amino-2-cyclohexenone and its yield can be increased further.

The above process can be performed either batchwise, semicontinuously or continuously. When 5-oxohexanenitrile is fully diluted with a solvent in the aforesaid process, the formation of the by-product 6-methyl-3,4-dihydro-2-pyridone can be inhibited to some extent, and the selectivity for 3-amino-2-cyclohexenone can be increased. Accordingly, it is preferred to use the solvent in an amount which is as excessive as is commercially permissible. It is preferred that the reaction be carried out under conditions such that the concentration of the starting 5-oxohexanenitrile in the reaction system does not exceed about 0.5 mole/liter, for example, under such conditions that the concentration of the 5-oxohexanenitrile is 0.1 to 0.5 mole/liter of solvent. The reaction time is not critical, either, and may, for example, be 0.5 to 4 hours.

The selectivity for 3-amino-2-cyclohexenone can thus be increased if 5-oxohexanenitrile is added to the reaction system so that the concentration of the unreacted 5-oxohexanenitrile in the reaction solution does not exceed 0.5 mole/liter.

After the reaction, the reaction mixture is treated in a customary manner such as distillation or crystallization to separate 3-amino-2-cyclohexenone.

Dehydrogenation of the resulting 3-amino-2-cyclohexenone in accordance with British Patent 1,481,573 can yield m-aminophenol. Furthermore, cyclohexane-1,3-dione can be obtained by reacting the 3-amino-2-cyclohexenone with water at a temperature of about 0 to about 200°C in the presence of a basic or acidic catalyst. Dehydrogenation of the cyclohexane-1,3-dione in accordance with, for example, British Patent 1,517,726 can afford resorcinol.

The process of the invention is specifically illustrated by the following examples.

Examples 1 to 9

An argon-purged 50 ml. stainless steel autoclave was charged with 5-oxohexanenitrile, the catalyst and the solvent shown in Table 1 in the amounts indicated, and the reaction was performed under the conditions shown in Table 1. After the reaction, the reaction mixture was analyzed by gas

chromatography, and the conversion of 5-oxohexanenitrile, and its selectivities for 3-amino-2-cyclohexenone and for 6-methyl-3,4-dihydro-2-pyridone were calculated. The results are shown in Table 1.

Comparative Examples 1 to 6

Example 1 was repeated except that the amount of 5-oxohexanenitrile, the catalyst, the solvent and the reaction conditions were changed as shown in Table 2. The results are shown in Table 2.

TABLE 1

| Example | Reaction solution | | | Reaction conditions | | Conversion of 5-oxohexanenitrile (%) | Selectivities for the products (%) | |
|---|---|---|---|---|---|---|---|---|
| | 5-oxohexanenitrile (mmoles) | Catalyst (mmoles) | Solvent (ml) | Temperature (°C) | Time (hrs) | | 3-amino-2-cyclohexanone | 6-methyl-3,4-dihydro-2-pyridone |
| 1 | 9.30 | tert-BuOK 0.85 | tert-BuOH 30 | 180 | 3 | 97 | 80 | 15 |
| 2 | 8.89 | ,, 0.87 | ,, 30 | 200 | 3 | 95 | 78 | 17 |
| 3 | 9.10 | ,, 0.88 | ,, 30 | 162 | 3 | 92 | 75 | 15 |
| 4 | 8.90 | ,, 0.85 | ,, 30 | 215 | 3 | 95 | 70 | 20 |
| 5 | 9.48 | ,, 0.91 | ,, 15 | 180 | 3 | 96 | 68 | 21 |
| 6 | 8.66 | ,, 0.91 | $Me_2EtCOH$ 30 | 180 | 3 | 95 | 78 | 16 |
| 7 | 3.19 | KOH 0.30 | tert-BuOH 30 | 180 | 3 | 95 | 87 | 13 |
| 8 | 8.85 | ,, 0.90 | ,, 30 | 180 | 3 | 97 | 79 | 17 |
| 9 | 8.64 | tert-BuONa 0.90 | ,, 50 | 180 | 3 | 88 | 71 | 16 |

TABLE 2

| Comparative Example | Reaction solution | | | Reaction conditions | | Conversion of 5-oxohexane-nitrile (%) | Selectivities for the products (%) | |
|---|---|---|---|---|---|---|---|---|
| | 5-oxohexanenitrile (mmoles) | Catalyst (mmoles) | Solvent (ml) | Temperature (°C) | Time (hrs) | | 3-amino-2-cyclohexanone | 6-methyl-3,4-dihydro-2-pyridone |
| 1 | 8.99 | tert-BuOK 0.90 | tert-BuOH 30 | 26 | 3 | 8 | 6 | 50 |
| 2 | 9.47 | ,, 0.90 | ,, 30 | 82 | 3 | 50 | 57 | 29 |
| 3 | 8.37 | ,, 0.73 | ,, 30 | 130 | 3 | 76 | 61 | 21 |
| 4 | 9.99 | ,, 0.88 | ,, 30 | 155 | 1 | 80 | 63 | 17 |
| 5 | 9.04 | ,, 0.97 | ,, 30 | 230 | 1 | 87 | 58 | 26 |
| 6 | 9.45 | tert-BuONa 0.90 | ,, 30 | 130 | 3 | 67 | 57 | 27 |

Examples 10 to 14

Example 1 was repeated except that a mixture of tert-butanol and a higher boiling non-polar aliphatic or aromatic hydrocarbon was used as the solvent, the amount of 5-oxohexanenitrile was changed as shown in Table 3, and potassium tert-butoxide in the amounts indicated in Table 3 was used as a catalyst.

The results are shown in Table 3.

TABLE 3

| Example | Reaction solution | | | Reaction conditions | | Conversion of 5-oxohexane-nitrile (%) | Selectivities for the products (%) | |
|---|---|---|---|---|---|---|---|---|
| | 5-oxohexanenitrile (mmoles) | Catalyst (mmoles) | Solvent (ml) | Temperature (°C) | Time (hrs) | | 3-amino-2-cyclo-hexanone | 6-methyl-3,4-dihydro-2-pyridone |
| 10 | 9.07 | tert-BuOK 0.86 | tert-BuOH (22) + toluene (8) | 180 | 3 | 95 | 78 | 15 |
| 11 | 8.80 | ,, 0.81 | tert-BuOH (15) + toluene (15) | 180 | 3 | 93 | 75 | 17 |
| 12 | 8.87 | ,, 0.89 | tert-BuOH (8) + toluene (22) | 180 | 3 | 85 | 70 | 18 |
| 13 | 9.65 | ,, 0.85 | tert-BuOH (15) + p-xylene (15) | 180 | 3 | 94 | 76 | 17 |
| 14 | 8.89 | ,, 0.87 | tert-BuOH (15) + n-octane (15) | 180 | 3 | 93 | 75 | 20 |

Example 15

A 50 ml stainless steel autoclave was charged with 0.41 mmole of potassium tert-butoxide, 3.09 mmoles of 5-oxohexanenitrile and 30 ml of tert-butanol. The mixture was heated at 180°C, and every 30 minutes, 2.81 mmoles of 5-oxohexanenitrile was freshly added. After heating for 1.5 hours, the reaction mixture was analyzed by gas chromatography. It was found that the conversion of 5-oxohexanenitrile was 74%, the selectivity for 3-amino-2-cyclohexenone was 87%, and the selectivity for 6-methyl-3,4-dihydro-2-pyridone was 10%.

Example 16

Example 15 was repeated except that the 5-oxohexanenitrile was added all at one time. Specifically, a mixture of 0.40 mmole of potassium tert-butoxide, 9.91 mmoles of 5-oxohexanenitrile and 30 ml of tert-butanol was heated at 180°C for 1.5 hours. The conversion of 5-oxohexanenitrile was 85%, the selectivity for 3-amino-2-cyclohexenone was 77%, and the selectivity for 6-methyl-3,4-dihydro-2-pyridone was 15%.

**Claims**

1. A process for producing a substituted cyclohexen-2-one by treating a cyanoethylated ketone with a basic catalyst in a polar solvent characterised in that 3-amino-2-cyclohexenone is produced by heating 5-oxohexanenitrile at 160°C to 220°C in the presence of a basic catalyst in an inert organic polar solvent.

2. A process according to claim 1 characterised in that the inert organic polar solvent is an alcohol of 1 to 6 carbon atoms, an ether of 4 to 14 carbon atoms, a nitrile of 1 to 3 carbon atoms, an amine of 3 to 8 carbon atoms, a dialkyl formamide wherein each alkyl group is of 1 or 2 carbon atoms, dimethyl sulfoxide, hexamethylphosphoric triamide, or a mixture of at least two of these.

3. A process according to claim 1 characterised in that the inert organic polar solvent is a mixture of a solvent as specified in claim 2 with an aliphatic or aromatic hydrocarbon of 6 to 10 carbon atoms.

4. A process according to claim 1 characterised in that the inert organic polar solvent is at least one tertiary alcohol of up to 6 carbon atoms or a mixture thereof with an aliphatic or aromatic hydrocarbon of 6 to 10 carbon atoms.

5. A process according to any one of the preceding claims characterised in that the basic catalyst is an alkali metal, alkali metal hydroxide, alkali metal alkoxide, alkali metal amide, alkali metal hydride or a mixture of at least two of these.

6. A process according to any one of the preceding claims characterised in that the concentration of the 5-oxohexanenitrile in the reaction system is up to 0.5 mole/liter.

7. A process according to any one of the preceding claims characterised in that the amount of the basic catalyst is 0.001 to 1 mole per mole of the 5-oxohexanenitrile.

**Revendications**

1. Procédé pour la préparation d'une cyclohexèn-2-one substituée par traitement d'une cétone cyano-éthylée avec un catalyseur basique dans un solvant polaire, caractérisé par le fait que la 3-amino-2-cyclohexénone est préparée en chauffant le 5-oxohexanenitrile à 160°—220°C en présence d'un catalyseur basique dans un solvant organique polaire, inerte.

2. Procédé selon la revendication 1, caractérisé par le fait que le solvant organique polaire, inerte, est un alcool ayant 1 à 6 atomes de carbone, un éther ayant 4 à 14 atomes de carbone, un nitrile ayant 1 à 3 atomes de carbone, une amine ayant 3 à 8 atomes de carbone, un dialkylformamide dans lequel chaque groupe alkyle a 1 à 2 atomes de carbone, le diméthylsulfoxyde, l'hexaméthylphosphoryltriamide, ou un mèlange d'au moins deux des ces solvants.

3. Procédé selon la revendication 1, caractérisé par le fait que le solvant organique polaire, inerte, est un mélange d'un solvant tel que défini dans la revendication 2 avec un hydrocarbure aliphatique ou aromatique ayant 6 à 10 atomes de carbone.

4. Procédé selon la revendication 1, caractérisé par le fait que le solvant organique polaire, inerte, est au moins un alcool tertiaire ayant jusqu'à 6 atomes de carbone, ou un mélange de cet alcool avec un hydrocarbure aliphatique ou aromatique ayant 6 à 10 atomes de carbone.

5. Procédé selon l'une quelconque des revendications précédentes, caractérisé par le fait que le catalyseur basique est un métal alcalin, un hydroxyde de métal alcalin, un alcoolate de métal alcalin, un amidure de métal alcalin, un hydrure de métal alcalin, ou un mélange d'au moins deux de ces produits.

6. Procédé selon l'une quelconque des revendications précédentes, caractérisé par le fait que la concentration du 5-oxohexanenitrile dans le système réactionnel va jusqu'à 0,5 mole/litre.

7. Procédé selon l'une quelconque des revendications précédentes, caractérisé par le fait que la quantité du catalyseur basique va de 0,001 à 1 mole par mole du 5-oxohexanenitrile.

**Patentansprüche**

1. Verfahren zur Herstellung eines substituierten Cyclohexen-2-ons durch Behandlung eines Cyanäthylierten Ketons mit einem basischen Katalysator in einem polaren Lösungsmittel, dadurch gekennzeichnet, daß 3-Amino-2-cyclohexenon hergestellt wird durch Erhitzen von 5-Oxohexannitril auf 160°C bis 220°C in Gegenwart eines basischen Katalysators in einem organischen polaren Lösungsmittel.

2. Verfahren nach Anspruch 1, dadurch gekennzeichnet, daß das inerte organische polare Lösungsmittel ein Alkohol mit 1 bis 6 Kohlenstoffatomen, ein Äther mit 4 bis 14 Kohlenstoffatomen, ein Nitril mit 1 bis 3 Kohlenstoffatomen, ein Amin mit 3 bis 8 Kohlenstoffatomen, ein Dialkylformamid, bei dem jede Alkylgruppe 1 bis 2 Kohlenstoffatome besitzt, Dimethylsulfoxid, Hexamethylphosphortriamid oder ein Gemisch aus mindestens zwei dieser Lösungsmittel ist.

3. Verfahren nach Anspruch 1, dadurch gekennzeichnet, daß das inerte organische polare Lösungsmittel ein Gemisch aus einem Lösungsmittel wie in Anspruch 2 angegeben, mit einem aliphatischen oder aromatischen Kohlenwasserstoff mit 6 bis 10 Kohlenstoffatomen ist.

4. Verfahren nach Anspruch 1, dadurch gekennzeichnet, daß das inerte organische polare Lösungsmittel mindestens ein tetriärer Alkohol mit bis zu 6 Kohlenstoffatomen oder ein Gemisch dieses Alkohols mit einem aliphatischen oder aromatischen Kohlenwasserstoffs mit 6 bis 10 Kohlenstoffatomen ist.

5. Verfahren nach einem der vorhergehenden Ansprüche, dadurch gekennzeichnet, daß der basische Katalysator ein Alkalimetall, Alkalihydroxid, Alkalialkoxid, Alkaliamid, Akalihydrid oder ein Gemisch aus mindestens zwei dieser Substanzen ist.

6. Verfahren nach einem der vorangehenden Ansprüche, dadurch gekennzeichnet, daß die Konzentration an 5-Oxohexannitril in dem Reaktionsgemisch bis zu 0,5 Mol/1 beträgt.

7. Verfahren nach einem der vorangehenden Ansprüche, dadurch gekennzeichnet, daß die Menge an basischem Katalysator 0,001 bis 1 Mol/Mol 5-Oxohexannitril beträgt.